Europäisches Patentamt

European Patent Office

Office européen des brevets

(19)

(11) Veröffentlichungsnummer: **0 282 727**
**A2**

(12) **EUROPÄISCHE PATENTANMELDUNG**

(21) Anmeldenummer: **88101910.3**

(22) Anmeldetag: **10.02.88**

(51) Int. Cl.⁴: **A61B 17/22**

(30) Priorität: **14.03.87 DE 3708325**

(43) Veröffentlichungstag der Anmeldung:
**21.09.88 Patentblatt 88/38**

(84) Benannte Vertragsstaaten:
**DE FR GB IT**

(71) Anmelder: **DORNIER MEDIZINTECHNIK GMBH**
**Postfach 1128**
**D-8034 Germering 1(DE)**

(72) Erfinder: **Heine, Gerold, Dr.**
**Reismühlenweg 7**
**D-7772 Uhldingen(DE)**
Erfinder: **Ganter, Doris**
**Atterseestrasse 12b**
**D-8000 München 60(DE)**

(74) Vertreter: **Landsmann, Ralf, Dipl.-Ing.**
**c/o DORNIER GMBH Postfach 1420**
**D-7990 Friedrichshafen 1(DE)**

(54) **Verbesserung der Chemolyse von Konkrementen.**

(57) Vorgestellt wird eine Verbesserung der Chemolyse von Konkrementen beispielsweise der Galle.
Durch akustische Wellen, Ultraschall oder Stosswellen, werden die Konkremente, oder Bruchstücke von
ihnen nach einer eventuell vorausgegangenen Zertrümmerung, in einem Lysemittel bewegt, um durch
die Bewegung und die erzeugte Erwärmung die
Auflösegeschwindigkeit der Konkremente zu
erhöhen.

Fig. 3

EP 0 282 727 A2

## Verbesserung der Chemolyse von Konkrementen

Die Erfindung betrifft die Verbesserung der Chemolyse von Konkrementen im Körper von Lebewesen.

Bei der chemischen Auflösung von Konkrementen, insbesondere von Gallensteinen, unterscheidet man zwischen der medikamentösen und der direkten Chemolyse. Bei der direkten Chemolyse wird das Lysemittel über einen Katheter in den Körper des Patienten eingeleitet. Gallengangsteine werden durch die ständige Perfusion des Lösungsmittels in die Gallengänge aufgelöst. Die Wahl des oder der Lösemittel richtet sich dabei nach der Zusammensetzung des Steines. Zur Zeit finden hauptsächlich Gallensalze EDTA, Monooctanoin und Methyltertiärbutyläther MTBE als Lösungsmittel Verwendung. In die Gallenblase wird das Lösemittel entweder perkutan oder retrograd über die Gallengänge eingebracht.

Aufgrund der Anwesenheit von Galleflüssigkeit ist ein Kontakt zwischen Stein und Lösungsmittel nicht unbedingt gewährleistet, da sich einige Lösungsmittel in ihren physikalischen Eigenschaften sehr von der Galleflüssigkeit unterscheiden und keine Mischung erfolgt. An den Grenzflächen zwischen Lösungsmittel und zu lösender Substanz stellen sich häufig gesättigte Lösungen ein, die einen weiteren Lösungsvorgang verhindern.

Um den Kontakt zwischen Lösungsmittel und Stein zu verbessern und somit die Auflösungsgeschwindigkeit zu erhöhen, wurden Verfahren entwickelt, die durch ständiges Infundieren und Aspirieren geringer Volumina an Lösungsmittel zu einer stetigen Umspülung des Steines führen sollen und eine Art Rühreffekt darstellen. Dieses Verfahren hat den Nachteil, dass ständig Lösungsmittel in geringen Mengen bewegt werden muss und die Spülwirkung bzw. der Kontakt zwischen Lösungsmittel und Stein stark von den fluiddynamischen Verhältnissen in der Gallenblase abhängt. Die sich ausbildenden Strömungen sind von den geometrischen Gegebenheiten der Blase, dem Katheter und der Infusions-bzw. Aspirationsgeschwindigkeit abhängig. Sie sind nicht kontrollierbar. Das Konkrement könnte zum Beispiel in einem Totwassergebiet liegen, so dass der Spülvorgang auf ihn keinen Einfluss ausübt. Diese liesse sich nur an der Behandlungszeit feststellen.

Der Erfindung liegt die Aufgabe zugrunde, die Auflösegeschwindigkeit eines Konkrements in einem geeigneten Lösemittel durch Erzielen einer Bewegung des Konkrements zu steigern, um damit die Behandlungszeit für einen Patienten zu verkürzen.

Die Aufgabe wird durch die erfindungsgemässe Vorrichtung nach Anspruch 1 und den weiteren Ansprüchen gelöst.

An den zu behandelnden Körperteil eines Patienten wird eine akustische Wellen erzeugende Einrichtung angekoppelt. Die Wellen durchdringen das Gewebe des Patienten ohne es zu beschädigen, und erreichen die Konkremente. Diese Konkremente zeichnen sich dadurch aus, dass ihre akustische Impedanz von der des Gewebes differiert. Der beim Auftreffen erfolgende Impedanzsprung erzeugt einen Reflexionsdruck am Konkrement, der eine rotatorische oder translatorische Bewegung hervorruft. Die Intensität der Bewegung und des darin bestehenden Rühreffektes der Konkremente im Lösungsmittel, welches sich beispielsweise in Gallengang oder Gallenblase um die Konkremente herum befindet, wobei sich die Wahl des Lösungsmittels nach der chemischen Zusammensetzung der Konkremente richtet, wird bestimmt durch die Schalleistung des beispielsweise verwendeten Ultraschallschwingers, die Periodendauer der Schallbeaufschlagung und die effektive Beschallungszeit. Der Ultraschallschwinger ist dabei auf das zu behandelnde Organ gerichtet und arbeitet entweder fokussiert oder unfokussiert. Er kann im Burst-Mode betrieben werden, das heisst,er arbeitet intermittierend. Durch die Bewegung der Konkremente in der Lyseflüssigkeit wird der Auflösevorgang deutlich beschleunigt, was in Verbindung mit der Temperaturerhöhung, die durch die vom Ultraschall hervorgerufene innere Reibung erzeugt wird, bei in-vitro-Versuchen zu einer Auflösesteigerung um den Faktor 10 führte. Die Bewegung ermöglicht auch, dass das Konkrement ständig mit frischer, weniger gesättigter Lyseflüssigkeit in Verbindung kommt, was der Auflösung ebenfalls förderlich ist. Die durch innere Reibung erzeugte Temperaturerhöhung ist ebenfalls über die Parameter Schalleistung, Periodendauer und effektive Beschallungszeit des Ultraschallschwingers steuerbar, es muss dabei aber darauf geachtet werden, dass die Temperatur nicht über den schädigenden Temperaturgrenzwert von 42° C ansteigt.

Anstatt der Ultraschallanlage ist auch die Verwendung einer stosswellenerzeugenden Einrichtung, beispielsweise einer Unterwasserfunkenstrecke, denkbar. Aber auch die Stosswellenerzeugung auf elektromagnetischem Wege, durch Piezo elemente oder mit Laser ist möglich. Die Stosswellen sind dabei fokussiert, teilfokussiert oder unfokussiert anwendbar. Hier ist eine durch innere Reibung erzeugte Temperaturerhöhung nicht möglich, jedoch der Rühreffekt ist erzielbar, wobei es zusätzlich möglich ist, durch fokussierte Stosswellen die aufzulösenden Konkremente vorher kleiner zu zertrümmern, was zu einer vergrösserten Oberfläche und damit zu einer vergrösserten Angriffsfläche für das Lösemittel führt, so dass dann die weitere Auflösung durch Lysemittel in erfindungsgemässer Vorrichtung fortgesetzt werden kann.

Die erfindungsgemässe Vorrichtung ermöglicht auf nichtinvasive Weise, den Prozess der Steinauflösung zu beschleunigen und damit die Behandlungszeit eines Patienten auf bis zu 1/10 der bisher benötigten Zeit zu reduzieren.

Denkbar ist ebenfalls, den Ultraschallschwinger einer Ultraschallortungseinrichtung so auszugestalten, dass er umschaltbar entweder zur Ortung oder zur Erzeugung der konkrementbewegenden Stosswellen dient.

Eine entsprechende Umschaltmöglichkeit wäre auch bei einer Stosswellenquelle mit Unterwasserfunkenstrecke möglich. Bei verschiedenen Druckverhältnissen kann die Stosswelleneinrichtung einmal Konkremente zertrümmern und zum anderen die Konkremente oder Konkrementbruchstücke bewegen.

Weitere Vorteile und Anwendungsmöglichkeiten ergeben sich aus den Figuren.

Es zeigen:

Figur 1 die prinzipielle Darstellung einer Einrichtung zur berührungsfreien Zerkleinerung und Bewegung von Konkrementen in Körpern von Lebewesen,

Figur 2 die prinzipielle Darstellung einer Einrichtung zur Bewegung von Konkrementen oder deren Bruchstücke,

Figur 3 die prinzipielle Darstellung eines Ultraschallschwingers in Auflage auf einem Körper und

Figur 4 die prinzipielle Darstellung einer Ausführungsform mit Stosswelleneinrichtung und Ultraschalleinrichtung in Kombination.

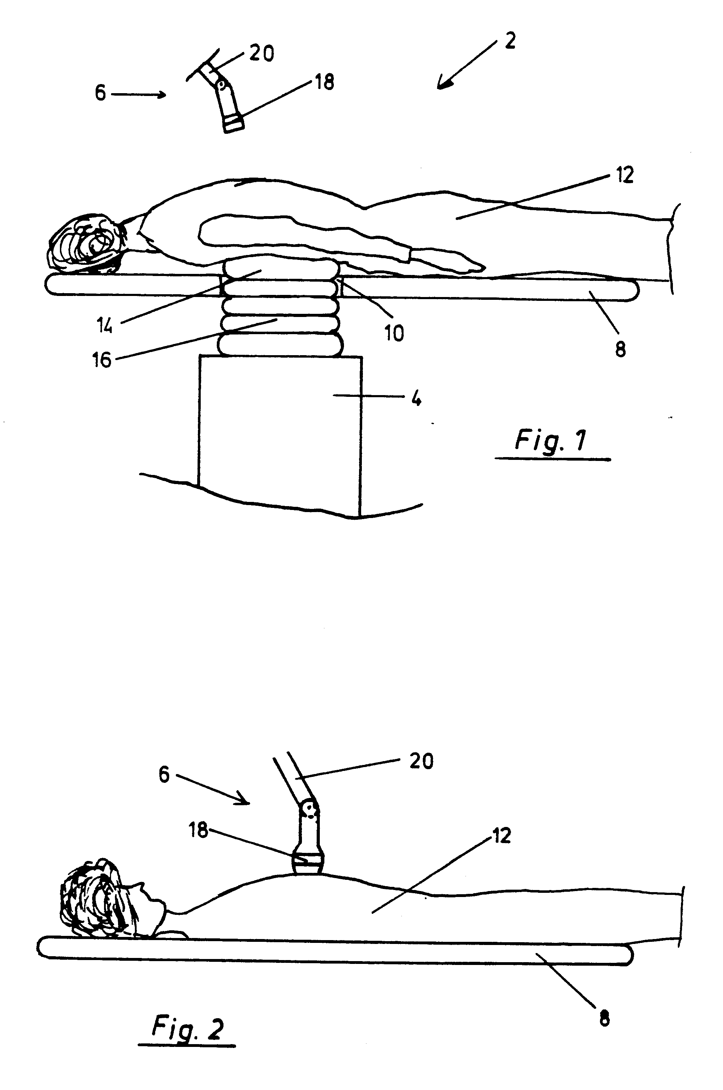

Figur 1 zeigt eine kombinierte Einrichtung 2 zur Zertrümmerung und Bewegung von Konkrementen und deren Bruckstücken im Körper eines Patienten mit einer Stosswelleneinrichtung 4 und einer Ultraschalleinrichtung 6.

Auf einer Patientenliege 8 mit einer Aussparung 10 befindet sich ein Patient 12 in Bauchlage, die bei der Behandlung von Gallenkonkrementen mit Stosswellen einzunehmen ist.

Durch die Aussparung 10 der Patientenliege 8 hindurch ragt ein Wasserkissen 14 mit Faltenbalg 16 der Stosswellen einrichtung 4 bis an den Körper des Patienten 12 heran und ist dort luftspalt-und luftblasenfrei, eventuell unter Verwendung eines Koppelgels, in dem Bereich angekoppelt, wo sich die Konkremente befinden. Durch die Stosswelleneinrichtung 4 können vorhandene Gallenkonkremente zunächst zertrümmert werden, so dass ihre Grösse verkleinert wird, gleichzeitig aber die Grösse ihrer Oberfläche zunimmt. Die Vergrösserung der Oberfläche wirkt sich positiv auf die nachfolgende weitere Auflösung der Konkremente durch Zuführung von Lysemittel aus.

Durch die Ultraschalleinrichtung 6 werden die Konkremente in beschriebener Form mit Ultraschall beaufschlagt. Dabei kann der Frequenzbereich zwischen 100 Hz und einigen MHz liegen. Erfolgreich wurde beispielsweise eine Frequenz von 200 kHz eingesetzt. Die Intensität des verwendeten Ultraschalls sollte dabei aus gewebeschonenden Aspekten im Bereich unterhalb von 20 W/cm² liegen.

Die Ultraschalleinrichtung besteht aus einem Ultraschallkopf 18 und einem oder mehreren frei beweglichen Armen 20, an die der Kopf 18 angeschlossen ist. Die Arme 20 können auch Teil einer hier nicht gezeigten, teleskopartigen Tragarmkonstruktion sein.

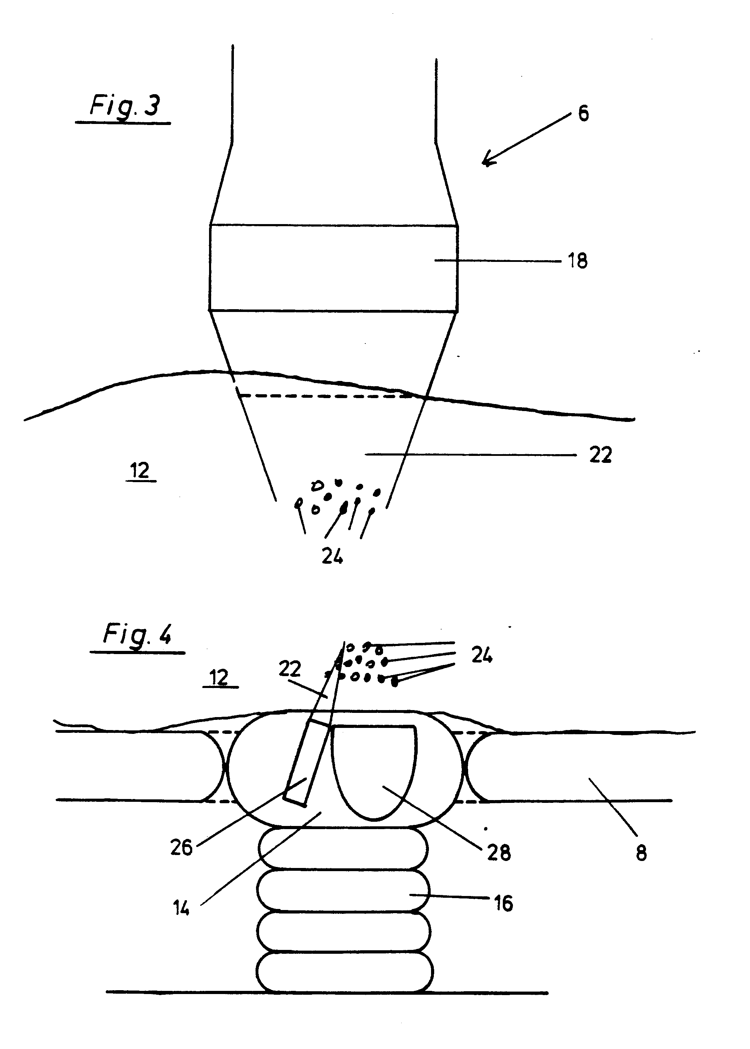

Wie auch aus Figur 2, die die Verwendung nur einer Ultra schalleinrichtung 6 zeigt, ersichtlich, wird der Ultraschallkopf 18 handgeführt etwa in den Bereich der zu behandelnden Organe, beispielsweise Gallengang oder Gallenblase, gebracht und kann dabei auch an dem Körper des Patienten 12 anliegen.

Figur 3 zeigt die Wirkungsweise des Ultraschalls; der aus dem Ultraschallkopf 18 austretende Schallkegel 22 muss dabei nicht notwendigerweise fokussiert werden. Schon eine grobe Ausrichtung des Schallkegels 22 auf einen gewissen Bereich, in dem sich die Konkremente 24 oder Bruchstücke davon befinden, genügt. Ein völlig unfokussierter Schallkegel wäre auch anwendbar, jedoch dient eien gewisse Fokussierung der Schonung des umgebenden Gewebes. Durch die auftreffenden Ultraschallwellen mit Druck-und Zugstufe werden die Konkremente 24 im Körperorgan in der eingeführten Lyseflüssigkeit bewegt wie oben beschrieben.

Figur 4 zeigt die Anwendungsmöglichkeit, dass Stosswelleneinrichtung 4 und Ultraschalleinrichtung 6 innerhalb des Wasserkissens 14 angeordnet sind. Dabei ist der Schallkegel 22 des Ultraschallschwingers 26 etwa auf den Punkt ausgerichtet, der den zweiten Brennpunkt eines Rotationsellipsoiden 28 bildet.

## Ansprüche

1. Verbesserung der Chemolyse von Konkrementen in Körpern von Lebewesen, **gekennzeichnet** durch eine an den Körper nahe der Konkremente angenäherte Vorrichtung, die ein Lysemittel und/oder die Konkremente mittels einer angepassten Druckwellenfront oder Stosswellenfront im Körper bewegt.

2. Vorrichtung zur Verbesserung der Chemolyse von Konkrementen in Körpern von Lebewesen nach Anspruch 1, **dadurch gekennzeichnet**, dass mehrere Wellenfronten aufeinanderfolgend ausgesendet werden.

3. Vorrichtung nach Ansprüchen 1 und 2, dadurch gekennzeichnet, dass mehrere Wellenfronten in gleicher zeitlicher Reihenfolge ausgesendet werden.

4. Vorrichtung nach Ansprüchen 1 bis 3, dadurch gekennzeichnet, dass die Vorrichtung eine Ultraschalleinrichtung ist.

5. Vorrichtung nach Ansprüchen 1 bis 3, dadurch gekennzeichnet, dass die Vorrichtung eine Stosswelleneinrichtung, insbesondere eine Unterwasserfunkenstrecke ist.

6. Vorrichtung nach Anspruch 4, dadurch gekennzeichnet, dass die Ultraschallwellen auf das zu behandelnde Organ ausgerichtet sind und fokussiert, teilfokussiert oder unfokussiert sind.

7. Vorrichtung nach Anspruch 5, dadurch gekennzeichnet, dass die Stosswellen auf das zu behandelnde Organ ausgerichtet sind und fokussiert oder unfokussiert sind.

8. Vorrichtung nach einem der vorherigen Ansprüche, dadurch gekennzeichnet, dass Frequenz, abgegebene Leistung und die Periodendauer zwischen zwei Energiezuführungen einstellbar sind.

*Fig. 1*

*Fig. 2*

Fig. 3

Fig. 4